(19) Europäisches Patentamt
European Patent Office
Office européen des brevets

(11) **EP 4 199 659 A1**

(12) **EUROPEAN PATENT APPLICATION**

(43) Date of publication:
**21.06.2023 Bulletin 2023/25**

(21) Application number: **21214350.7**

(22) Date of filing: **14.12.2021**

(51) International Patent Classification (IPC):
*H05G 1/26* $^{(2006.01)}$          *G01N 23/083* $^{(2018.01)}$
*A61B 6/00* $^{(2006.01)}$          *G06T 11/00* $^{(2006.01)}$

(52) Cooperative Patent Classification (CPC):
**H05G 1/265; A61B 6/40; A61B 6/482;**
**G01N 23/083; G06T 11/005; H05G 1/58;**
A61B 6/032; A61B 6/52

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventor: **PROKSA, Roland
Eindhoven (NL)**

(74) Representative: **Philips Intellectual Property &
Standards
High Tech Campus 52
5656 AG Eindhoven (NL)**

(54) **APPARATUS FOR DETERMINING AN EFFECTIVE SPECTRUM OF AN X-RAY TUBE AND HIGH VOLTAGE GENERATOR FOR SPECTRAL X-RAY IMAGING**

(57)     The present invention relates to X-ray imaging. In order to improve the imaging performance of an X-ray imaging system, an apparatus is provided that comprises an input unit, a processing unit, and an output unit. The input unit is configured to receive the tube voltage and an emission current of the X-ray tube for successive measurement intervals. For each measurement interval, the processing unit is configured to divide the respective measurement interval into a predetermined number of time intervals at a temporal sampling frequency, extract a plurality of emission current samples from the received emission current at the temporal sampling frequency, discretize the received tube voltage to a finite set of discrete tube voltages, determine a current-density-function for each discrete tube voltage assignment, which represents accumulated current-time-products of emission current samples having the respective discrete tube voltage assignment, and determine the effective spectrum of the X-ray tube for the respective measurement interval as a function of the finite set of discrete tube voltages and the determined current-density-function for each discrete tube voltage assignment, and provide, via the output unit, the determined effective spectrum of the X-ray tube for the respective measurement interval.

Fig. 2

EP 4 199 659 A1

## Description

FIELD OF THE INVENTION

The present invention relates to X-ray imaging, and in particular to an apparatus, a method, and a system for determining an effective spectrum of an X-ray tube and high voltage generator for spectral X-ray imaging that switches a tube voltage between successive measurement intervals, to an X-ray imaging system and method, to a computer program, and to a computer readable medium.

BACKGROUND OF THE INVENTION

High voltage generator for X-ray imaging are typically designed to provide a constant output voltage with minimal ripple (i.e. noise). These generators typically use low pass filtration (e.g. smoothing capacitors) to reduce unwanted variations/ripple.

Rapid kVp-switching (kVp-S) is a spectral imaging technique that switches the voltage (kVp) rapidly between successive measurement intervals to obtain spectral information. A theoretically optimal kVp-S generator should switch infinitely fast and should have small waveform ripple. This however can hardly be realized. One important design parameter for the generator is the strength of the smoothing with conflicting impact on the switching speed and the ripple. Strong smoothing provides small ripple but slow transitions, and vice versa. However, strong smoothing may also have impacts to the final imaging performance of an X-ray imaging system. For example, slow switching speed may not allow for fast imaging especially for low dose protocols. If the transition times from low to high kVp (and back) become long, the spectral separation will decrease because the effective difference of the two spectra will diminish. For example, a strong ripple may change the effective output spectrum for the integration periods relative to the ideal trapezoidal waveform. If this difference becomes significant and cannot be corrected somehow, the results of the material decomposition, done to extract the clinically relevant information from the spectral acquisition, will be corrupted.

SUMMARY OF THE INVENTION

There may, therefore, be a need for improvement of the imaging performance of an X-ray imaging system.

The object of the present invention is solved by the subject-matter of the appended independent claims, wherein further embodiments are incorporated in the dependent claims.

According to a first aspect of the present invention, there is provided an apparatus for determining an effective spectrum of an X-ray tube and high voltage generator for spectral X-ray imaging that switches a tube voltage between successive measurement intervals. The apparatus comprises an input unit, a processing unit, and an output unit. The input unit is configured to receive the tube voltage and an emission current of the X-ray tube for successive measurement intervals. For each measurement interval, the processing unit is configured to divide the respective measurement interval into a predetermined number of time intervals at a temporal sampling frequency, extract a plurality of emission current samples from the received emission current at the temporal sampling frequency, discretize the received tube voltage to a finite set of discrete tube voltages, determine a current-density-function for each discrete tube voltage assignment, which represents accumulated current-time-products of emission current samples having the respective discrete tube voltage assignment, and determine the effective spectrum of the X-ray tube for the respective measurement interval as a function of the finite set of discrete tube voltages and the determined current-density-function for each discrete tube voltage assignment, and provide, via the output unit, the determined effective spectrum of the X-ray tube for the respective measurement interval.

The apparatus and method as described herein use minimal smoothing to enable fast imaging and to correct for the impact of strong ripple by measuring the effective spectrum and use this information for the image processing (e.g. material decomposition). The apparatus and method as described herein also provide a measurement technique able to provide the effective spectra for kVp switching applicable for X-ray imaging systems with particular focus on spectral CT imaging.

Additionally, higher kV ripple may be acceptable without the disadvantage of unknown spectral input signals for the data acquisition per detector frame. This would allow for low-capacitance systems with faster switching times and even for imperfect switching performance and/or voltage overshoot/under shoot situations.

Further, the voltage generator may be simplified by reducing the stringent specification in terms of regulation. This may reduce the requirements of in terms of having the rise/fall times as independent as possible to the operation point of the voltage generator.

In some examples, the apparatus as described herein may be used in medical X-ray imaging or image display. For example, the apparatus may be used in C-arm or CT X-ray systems utilizing spectral information within the X-rays. In some examples, the apparatus as described herein may be applicable to non-medical X-ray imaging or image display. For example, the apparatus may be used in non-destructive testing systems or in screening systems, e.g., airport luggage screening systems. In some further examples, the apparatus may be used in tomosynthesis imaging or image display.

According to an example, the processing unit may be configured to obtain a plurality of current-density-functions for each discrete tube voltage assignment from a plurality of measurement intervals having an identical energy level. The processing unit may be configured to average the plurality of current-density-functions to obtain a mean effective current-density-function for the respective discrete tube voltage assignment. The processing unit may be configured to determine the effective spectrum of the X-ray tube for the respective energy level as a function of the finite set of discrete tube voltages and the determined mean effective current-density-function for each discrete tube voltage assignment.

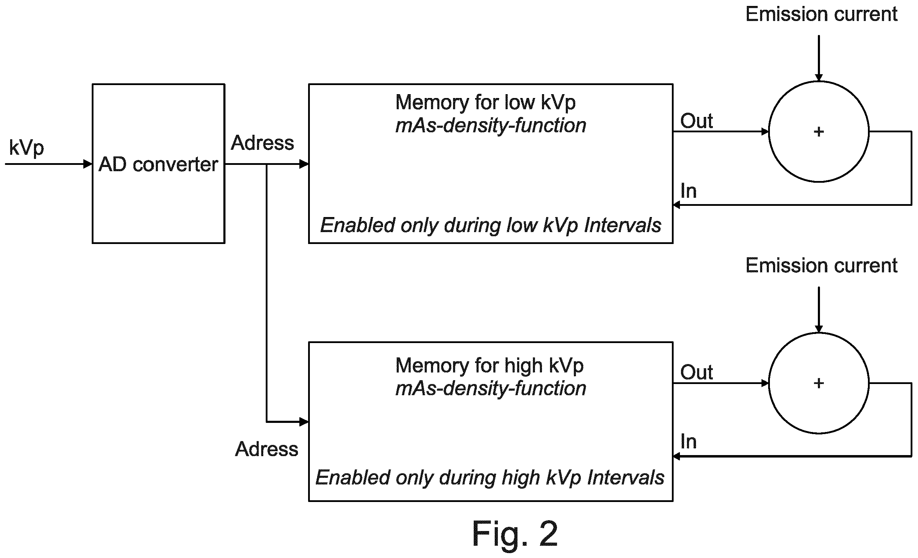

In this way, the effort for the data processing can be reduced. If the material decomposition is based on a look-up-table (LUT), the effort to generate the LUT would be strongly reduced. This will be explained hereinafter and in particular with respect to the example shown in Fig. 2.

According to an example, the emission current may be a known function of the tube voltage. The processing unit may be configured to determine the current-density-function for each discrete tube voltage assignment as a function of a number of occurrences of the respective discrete tube voltage assignment.

In this way, the current-density-function can be further simplified, and the effort for the data processing can be reduced.

According to an example, the tube voltage is obtainable at an operating point of the X-ray tube and high voltage generator at factory and/or during X-ray source conditioning.

In an example, the acquisition of the tube voltage may be done during regular operation.

In another example, the acquisition of the tube voltage may be done at factory.

In a further example, the acquisition of tube voltage may be done during tube conditioning, such as regular tube conditioning, to account for potential drifts over time or environmental conditions.

According to a second aspect of the present invention, there is provided a system for determining an effective spectrum of an X-ray tube for spectral X-ray imaging that switches a tube voltage between successive measurement intervals. The system comprises a voltage acquisition device configured to acquire a tube voltage of an X-ray tube, a current acquisition device configured to acquire an emission current of the X-ray tube, and an apparatus for determining an effective energy spectrum of the X-ray tube based on the measured tube voltage and the measured emission current according to the first aspect and any associated example.

According to a third aspect of the present invention, there is provided an X-ray imaging system. The X-ray imaging system comprises an image acquisition apparatus comprising an X-ray tube, a system for determining an effective spectrum of the X-ray tube according to the second aspect and any associated example, and a reconstruction apparatus. The image acquisition apparatus is configured to scan an object of interest to generate X-ray image data comprising one or more image slices, each representative of a certain thickness of the object being scanned in a certain angle of projection. The spectral correction system is configured to output an effective spectrum of the X-ray tube. The reconstruction apparatus is configured to apply the effective spectrum of the X-ray tube for image reconstruction.

According to a fourth aspect of the present invention, there is provided a method for determining an effective spectrum of an X-ray tube for spectral X-ray imaging that switches a tube voltage between successive measurement intervals. The method comprises:

- receiving the tube voltage and an emission current of the X-ray tube for the plurality of successive measurement intervals;
- dividing each measurement interval into a predetermined number of time intervals at a temporal sampling frequency;
- extracting a plurality of emission current samples from the received emission current at the temporal sampling frequency;
- discretizing the received voltage to a finite set of discrete tube voltages;
- determining a current-density-function for each discrete tube voltage assignment, which represents accumulated current-time-products of emission current samples having the respective discrete tube voltage assignment; and
- determining the effective spectrum of the X-ray tube for the respective measurement interval as a function of the finite set of discrete tube voltages and the determined current-density-function for each discrete tube voltage assignment.

According to an example, the method may further comprise:

- obtaining a plurality of current-density-functions for each discrete tube voltage assignment from a plurality of measurement intervals having an identical energy level;
- averaging the plurality of current-density-functions to obtain a mean effective current-density-function for the respective discrete tube voltage assignment; and
- determining the effective spectrum of the X-ray tube for the respective energy level as a function of the finite set of discrete tube voltages and the determined mean effective current-density-function for each discrete tube voltage assignment.

According to an example, the emission current may be a known function of the tube voltage. The current-density-function for each discrete tube voltage assignment may be determined as a function of a number of occurrences of the respective discrete tube voltage assignment.

According to an example, the tube voltage may be obtained at an operating point of the X-ray source at factory and/or during X-ray source conditioning.

According to a fourth aspect of the present invention, there is provided an X-ray imaging method, comprising:

- scanning an object of interest to generate X-ray image data comprising one or more image slices, each representative of a certain thickness of the object being scanned in a certain angle of projection;
- providing an effective spectrum of an X-ray tube according to the third aspect and any associated example; and
- applying the effective spectrum of the X-ray tube for image reconstruction.

The method may be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise of one or more processors, a memory, a data interface, or the like.

According to another aspect of the present invention, there is provided a computer program for controlling an apparatus according to the first aspect, which when executed by a processor is configured to carry out the method according to the third aspect, and/or a computer program for controlling a system according to the second aspect, which when executed by a processor is configured to carry out the method of the fourth aspect.

According to another aspect of the present invention, there is provided a computer readable medium having stored the program element.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiments) described hereinafter.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.

Fig. 1 illustrates an exemplary apparatus for determining the current-density-function of an X-ray tube and a high voltage generator for spectral X-ray imaging that switches a tube voltage between successive measurement intervals.

Fig. 2 illustrates an exemplary implementation of current-density-function measurement.

Fig. 3 illustrates a system for determining an effective spectrum of an X-ray tube for spectral X-ray imaging that switches a tube voltage between successive measurement intervals.

Fig. 4 illustrates an exemplary X-ray imaging system.

Fig. 5 illustrates a flow chart describing an exemplary method for determining an effective spectrum of an X-ray tube for spectral X-ray imaging that switches a tube voltage between successive measurement intervals.

Fig. 6 illustrates a flow chart describing an exemplary X-ray imaging method.

DETAILED DESCRIPTION OF EMBODIMENTS

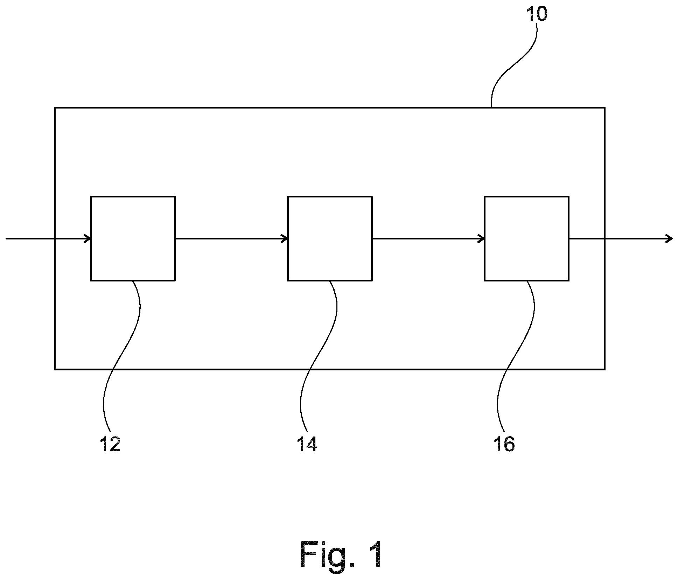

Fig. 1 illustrates an exemplary apparatus 10 for determining an effective spectrum of an X-ray tube and a high voltage generator for spectral X-ray imaging that switches a tube voltage between successive measurement intervals. The exemplary apparatus 10 comprises an input unit, 12, one or more processing unit 14, and an output unit 16.

In general, the apparatus 10 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although Fig. 1 may show a limited number of components by way of example, it can be appreciated that a greater or a fewer number of components may be employed for a given implementation.

In some implementations, the apparatus 10 may be embodied as, or in, a device or apparatus, such as a server, workstation, or mobile device. The apparatus 10 may comprise one or more microprocessors or computer processors, which execute appropriate software. The processing unit 14 of the apparatus 10 may be embodied by one or more of these processors. The software may have been downloaded and/or stored in a corresponding memory, e.g., a volatile memory such as RAM or a non-volatile memory such as flash. The software may comprise instructions configuring the one or more processors to perform the functions as described herein.

It is noted that the apparatus 10 may be implemented with or without employing a processor, and also may be implemented as a combination of dedicated hardware to perform some functions and a processor (e.g.,

one or more programmed microprocessors and associated circuitry) to perform other functions. For example, the functional units of the apparatus 10, e.g., the input unit 12, the one or more processing units 14, and the output unit 16 may be implemented in the device or apparatus in the form of programmable logic, e.g., as a Field-Programmable Gate Array (FPGA). In general, each functional unit of the apparatus may be implemented in the form of a circuit.

In some implementations, the apparatus 10 may also be implemented in a distributed manner. For example, some or all units of the apparatus 10 may be arranged as separate modules in a distributed architecture and connected in a suitable communication network, such as a 3rd Generation Partnership Project (3GPP) network, a Long Term Evolution (LTE) network, Internet, LAN (Local Area Network), Wireless LAN (Local Area Network), WAN (Wide Area Network), and the like.

The input unit 12 is configured to receive the tube voltage generated by the voltage generator that provides electrical input power into an X-ray tube, and an emission current of the X-ray tube for successive measurement intervals. The tube voltage may be acquired during regular operation, e.g. by measuring the voltage at the output of the voltage generator. Alternatively, the tube voltage may be measured at factory or during tube conditioning.

Each measurement interval may also be referred to as integration period (IP), within which a particular tube voltage is applied. We name the accumulated X-ray spectrum for one IP the effective spectrum. There are several non-varying impacts to the spectrum, such as filtration, heel effect, etc., which are not of interest in this context and are summarized with an effective filtration $F_E$ for the discussion. The two important parameters here are the tube voltage and the emission current in the X-ray tube. Since the output spectrum scales linear with the emission current, we model the spectrum with:

$$S_{t,E} = I_t F_E T_E(V_t)$$

where $S_{t,E}$ is the energy and time dependent output spectrum, $I_t$ is the emission current, and $T_E$ is the voltage and energy dependent tube output spectrum for the tube voltage $V_t$. The effective output spectrum for one IP becomes:

$$\tilde{S}_E = F_E \int_0^{t_{IP}} I_t T_E(V_t) dt$$

We discretize the model in the time domain and represent it via K discrete samples. In addition, we discretize the tube voltages to a finite set

$$V_i \leftarrow \ddot{V}_j \in \{\ddot{V}_1, \dots \ddot{V}_m\}$$

Now we reorganize the sum into the voltage bins $\{V_1, \dots V_m\}$:

$$\hat{S}_E = F_E \sum_{j=1}^{m} T_E(V_j) \sum_{V_i \leftarrow \ddot{V}_j} \Delta_{t,i} I_i$$

The inner $m$ sums are the accumulated current-time-products *(mAs)* of all time samples having the same discrete tube voltage $V_j$ assignment. We call this function $D(V_j)$ the mAs-density-function or current-density-function. Everything but the inner sum is constant and does not depend on the dynamic generator behavior.

The apparatus 10 as described herein is provided to measure the current-density-function (i.e. mAs-density-function) in the generator and to use this function to estimate the effective spectrum $\hat{S}_E$ used in the processing.

In particular, the processing unit 14 is configured to divide one IP into a predetermined number of time intervals at a temporal sampling frequency, and extract a plurality of emission current samples (e.g. K discrete samples) from the received emission current at the temporal sampling frequency. The processing unit 14 is further configured to discretize the received tube voltage to a finite set of discrete tube voltages, such as $\{V_1, \dots V_m\}$ as mentioned above.

The processing unit 14 is further configured to determine a current-density-function for each discrete tube voltage assignment, which represents accumulated current-time-products of emission current samples having the respective discrete tube voltage assignment. In this way, we can calculate the current-density-function $D(V_j)$ with:

$$D(V_j) = \sum_{V_i \leftarrow \ddot{V}_j} \Delta_{t,i} I_i$$

Finally, the processing unit 14 is configured to determine the effective spectrum of the X-ray tube for the respective measurement interval as a function of the finite set of discrete tube voltages and the determined current-density-function for each discrete tube voltage assignment using the following function:

$$\hat{S}_E = F_E \sum_{j=1}^{m} T_E(V_j) D(V_j)$$

The output unit 16 is configured to provide the determined effective spectrum of the X-ray tube for the respective measurement interval, which is preferably usable for image reconstruction (e.g. material decomposition).

In some examples, the time intervals $\Delta_{t,i}$ may be usually constant and may be moved out of the sum such that we can model the effective output spectrum with:

$$\hat{S}_E = \Delta_t F_E \sum_{j=1}^{m} T_E(V_j) \sum_{V_i \leftarrow \bar{V}_j} I_i$$

If the emission current is known to be a function of the tube voltage $I = F(V)$, the above equation may be further be simplified to:

$$\hat{S}_E = \Delta_t F_E \sum_{j=1}^{m} T_E(V_j) F(V_i) \sum_{V_i \leftarrow \bar{V}_j} 1$$

The inner sums become the number of occurrences (i.e. histogram) of the tube voltage $V_j$. In this case, the processing unit 14 may be configured to determine he current-density-function for each discrete tube voltage assignment as a function of a number of occurrences of the respective discrete tube voltage assignment multiplied by the known emission current function F(V).

Rapid kVp switching CT typically acquires two or more successive measurement intervals. For the data processing, it may be necessary to know the effective spectra for each measurement interval or for each type of measurement interval (e.g. low kVp or high kVp). Based on the stability and reproducibility of the effective spectra different operations can be used.

In some examples, for each image acquisition and IP, the current-density-function (i.e. mAs-density-function) is measured. The results are individually used of each IP.

In some examples, for each image acquisition comprising several measurement intervals, the current-density-function is measured and the results of identical measurement interval types (e.g. low kVp, high kVp) is averaged to obtain a mean effective current-density-function. In this way, the effort for the data processing can be reduced. If the material decomposition is based on a look-up-table (LUT), the effort to generate the LUT would be strongly reduced.

Fig. 2 illustrates an exemplary implementation of current-density-function measurement. In this example, two memories are used to accumulate and store the mAs-density-functions. The memory address is generated from a discrete representation of the actual kVp. For each cycle, the content of the memory cell addressed by the actual kVp is incremented with the actual emission current. The results of low kVp are averaged to obtain a mean effective current-density-function for the low kVp, and the results of high kVp are averaged to obtain a mean effective current-density-function for the high kVp.

In some examples, if the reproducibility of the mAs-density-function of the generator is high enough, the mAs-density-function measurement may be done in a calibration procedure (e.g. once a day or week). This would further reduce the effort for a LUT based material decomposition.

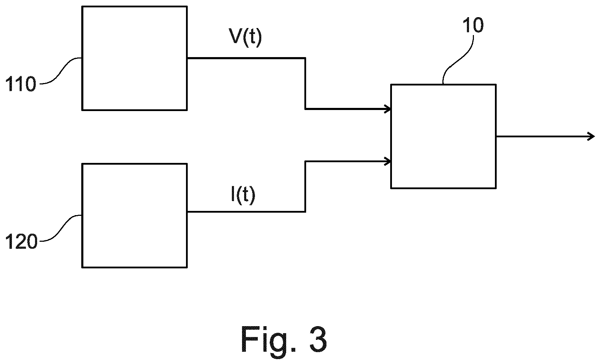

Fig. 3 shows a system 100 for determining an effective spectrum of an X-ray tube for spectral X-ray imaging that switches a tube voltage between successive measurement intervals. The spectral correction system 100 comprises a voltage acquisition device 110, a current acquisition device 120, and an apparatus 10 similar to the apparatus 10 shown in Fig. 1.

The voltage acquisition device 110 is configured to measure the tube voltage generated by a voltage generator that provides electrical input power into the X-ray tube. For example, the voltage acquisition device 110 may measure the voltage signal at the output of the voltage generator, by means of a voltage divider.

The current acquisition device 120 is configured to measure an emission current of the X-ray tube.

The apparatus 10 is configured to determine an effective energy spectrum of the X-ray tube based on the measured tube voltage and the measured emission current according to the method as described herein. The determined effective energy spectrum of the X-ray tube may be usable for image reconstruction.

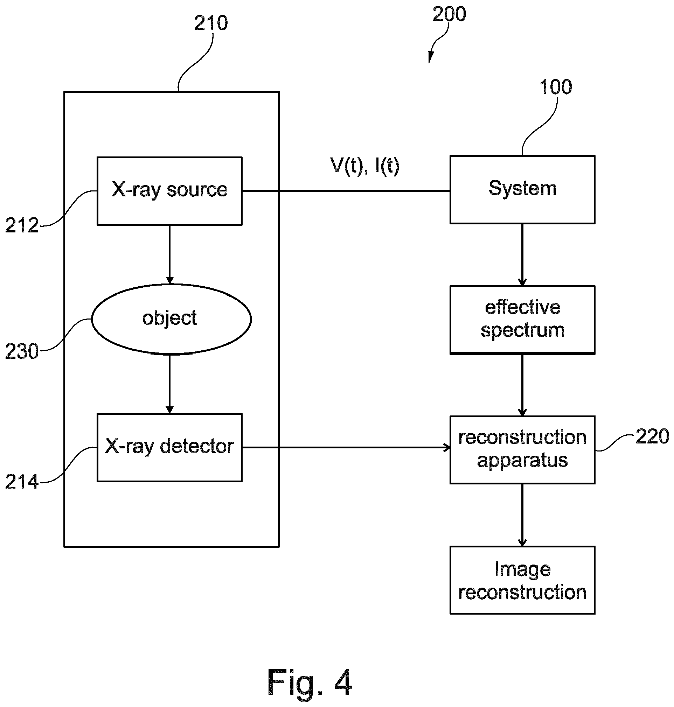

Fig. 4 shows an exemplary X-ray imaging system 200 according to some embodiments of the present disclosure. Examples of the X-ray imaging system may include, but are not limited to, a C-arm system, a computed tomography (CT) system, a digital X-ray radiography (DXR) system, and an image-guided therapy (IGT) system.

The X-ray imaging system 200 comprises an image acquisition apparatus 210, a 100 according to the system described with respect to Fig. 3, and a reconstruction apparatus 220.

The image acquisition apparatus 210 comprises an X-ray detector 214 opposing the X-ray source 212. The image acquisition apparatus 210 is configured to scan an object of interest 230 to generate X-ray image data, namely raw-image, comprising one or more image slices, each representative of a certain thickness of the object being scanned in a certain angle of projection.

The system 100 is configured to obtain a tube voltage generated by a voltage generator that provides electrical input power into the X-ray source. The system 100 is further configured to obtain an emission current of the X-ray tube. The system 100 is configured to output an effective spectrum of the X-ray source per measurement interval or per type of measurement interval (e.g. low kVp or high kVp).

The reconstruction apparatus 220 is configured to apply the effective spectrum of the X-ray tube for image reconstruction, which may include various image types including, but not limited to, weighted average images (simulating single energy spectra), virtual monoenergetic images (attenuation at a single photon energy rather than a spectrum), material decomposition images (mapping or removing substances of known attenuation characteristics, such as iodine, calcium, or uric acid), virtual non-contrast images (iodine removed), iodine concentration (iodine maps), calcium suppression (calcium removed), uric acid suppression (uric acid removed), electron density maps, and effective atomic number ($Z_{eff}$) maps.

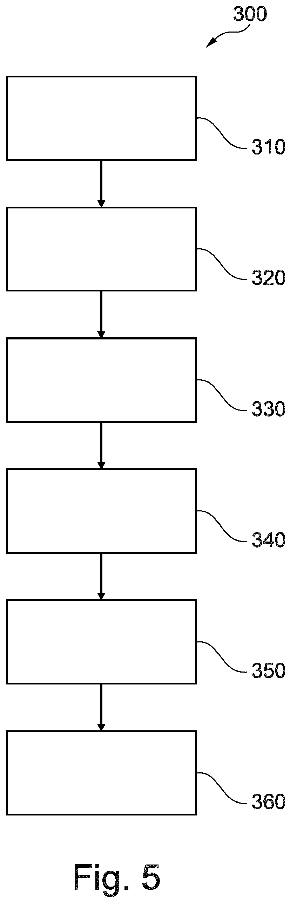

Fig. 5 illustrates a flow chart describing an exemplary method 300 according to an embodiment of the present disclosure.

The method 300 may be implemented as a device, module or related component in a set of logic instructions stored in a non-transitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 200 may be written in any combination of one or more programming languages, including an object oriented programming language such as JAVA, SMALLTALK, C++, Python, or the like and conventional procedural programming languages, such as the "C" programming language or similar programming languages. For example, the exemplary method may be implemented as the apparatus 10 shown in Fig. 1.

At block 310, the tube voltage and an emission current of the X-ray tube for the plurality of successive measurement intervals are received. The tube voltage and the emission current may be measured using a voltage acquisition device and a current acquisition device.

At block 320, each measurement interval is divided into a predetermined number of time intervals at a temporal sampling frequency.

At block 330, a plurality of emission current samples are extracted from the received emission current at the temporal sampling frequency. The emission current may be properly sampled and processed using standard integrated circuits, such as ADCs and FPGAs.

At block 340, the received voltage is discretized to a finite set of discrete tube voltages. For example, we discretize the tube voltages to a finite set

$$V_i \leftarrow \ddot{V}_j \in \left\{ \ddot{V}_1, \dots \ddot{V}_m \right\}.$$

At block 350, a current-density-function for each discrete tube voltage assignment is determined, which represents accumulated current-time-products of emission current samples having the respective discrete tube voltage assignment. For example, we model the current-density-function $D(V_j)$ with:

$$D\left(V_j\right) = \sum_{V_i \leftarrow \ddot{V}_j} \Delta_{t,i} I_i$$

where $I_i$ is the emission current of the $i_{th}$ discrete sample, and $\Delta_{t,i}$ is the time interval of the $i_{th}$ discrete sample.

At block 360, the effective spectrum of the X-ray tube for the respective measurement interval is determined as a function of the finite set of discrete tube voltages and the determined current-density-function for each discrete tube voltage assignment. For example, we can model the effective spectrum with:

$$\hat{S}_E = F_E \sum_{j=1}^{m} T_E(V_j)D(V_j)$$

where $S_E$ is the energy dependent output spectrum, $T_E$ is the voltage and energy dependent tube output spectrum for the tube voltage $V_j$, and $F_E$ is the effective filtration.

In some examples, the method 300 may further comprise the steps of obtaining a plurality of current-density-functions for each discrete tube voltage assignment from a plurality of measurement intervals having an identical energy level, averaging the plurality of current-density-functions to obtain a mean effective current-density-function for the respective discrete tube voltage assignment, and determining the effective spectrum of the X-ray tube for the respective energy level as a function of the finite set of discrete tube voltages and the determined mean effective current-density-function for each discrete tube voltage assignment. These steps are discussed with respect to the example shown in Fig. 2.

In some examples, the emission current may be a known function of the tube voltage, e.g. $I = F(V)$. The current-density-function for each discrete tube voltage assignment may be determined as a function of a number of occurrences of the respective discrete tube voltage assignment. For example, we can model the current-density-function $D(V_j)$ with:

$$D\left(V_j\right) = F(V_i) \sum_{V_i \leftarrow \ddot{V}_j} 1$$

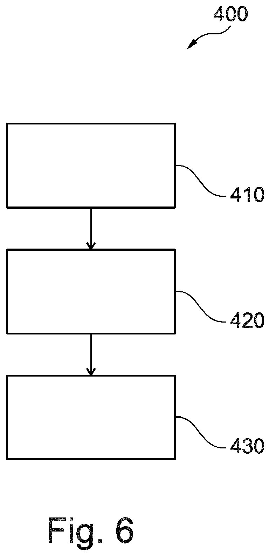

Fig. 6 illustrates a flow chart describing an exemplary X-ray imaging method 400. At block 410, an object of interest is scanned to generate X-ray image data comprising one or more image slices, each representative of a certain thickness of the object being scanned in a certain angle of projection.

At block 420, an effective spectrum of an X-ray tube can be determined according to the method described herein, e.g. the example shown in Fig. 4.

At block 430, the effective spectrum of the X-ray tube is applied for image reconstruction.

In another exemplary embodiment of the present invention, a computer program or a computer program element is provided that is characterized by being adapted to execute the method steps of the method according to one of the preceding sfs45kk, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. An apparatus (10) for determining an effective spectrum of an X-ray tube and high voltage generator for spectral X-ray imaging that switches a tube voltage between successive measurement intervals, the apparatus comprising:

   - an input unit (12); and
   - a processing unit (14); and
   - an output unit (16);

      wherein the input unit is configured to receive the tube voltage and an emission current of the X-ray tube for successive measurement intervals;
      wherein, for each measurement interval, the processing unit is configured to:

   - divide the respective measurement interval into a predetermined number of time intervals at a temporal sampling frequency;
   - extract a plurality of emission current samples from the received emission current at the temporal sampling frequency;
   - discretize the received tube voltage to a finite set of discrete tube voltages;
- determine a current-density-function for each discrete tube voltage assignment, which represents accumulated current-time-products of emission current samples having the respective discrete tube voltage assignment; and
- determine the effective spectrum of the X-ray tube for the respective measurement interval as a function of the finite set of discrete tube voltages and the determined current-density-function for each discrete tube voltage assignment; and
- provide, via the output unit, the determined effective spectrum of the X-ray tube for the respective measurement interval.

2. The apparatus according to claim 1,

wherein the processing unit is configured to obtain a plurality of current-density-functions for each discrete tube voltage assignment from a plurality of measurement intervals having an identical energy level;
wherein the processing unit is configured to average the plurality of current-density-functions to obtain a mean effective current-density-function for the respective discrete tube voltage assignment; and
wherein the processing unit is configured to determine the effective spectrum of the X-ray tube for the respective energy level as a function of the finite set of discrete tube voltages and the determined mean effective current-density-function for each discrete tube voltage assignment.

3. The apparatus according to claim 1 or 2,

wherein the emission current is a known function of the tube voltage; and
wherein the processing unit is configured to determine the current-density-function for each discrete tube voltage assignment as a function of a number of occurrences of the respective discrete tube voltage assignment.

4. The apparatus according to any one of the preceding claims,
wherein the tube voltage is obtainable at an operating point of the X-ray tube and high voltage generator at factory and/or during X-ray source conditioning.

5. A system (100) for determining an effective spectrum of an X-ray tube for spectral X-ray imaging that switches a tube voltage between successive measurement intervals, comprising:

- a voltage acquisition device (110) configured to acquire a tube voltage of an X-ray tube;
- a current acquisition device (120) configured to acquire an emission current of the X-ray tube; and
- an apparatus for determining an effective energy spectrum of the X-ray tube based on the measured tube voltage and the measured emission current according to any one of the preceding claims.

6. An X-ray imaging system (200), comprising;

- an image acquisition apparatus (210) comprising an X-ray tube;
- a system (100) for determining an effective spectrum of the X-ray tube according to claim 5; and
- a reconstruction apparatus (220);

wherein the image acquisition apparatus is configured to scan an object of interest to generate X-ray image data comprising one or more image slices, each representative of a certain thickness of the object being scanned in a certain angle of projection;
wherein the spectral correction system is configured to output an effective spectrum of the X-ray tube; and
wherein the reconstruction apparatus is configured to apply the effective spectrum of the X-ray tube for image reconstruction.

7. A method (300) for determining an effective spectrum of an X-ray tube for spectral X-ray imaging that switches a tube voltage between successive measurement intervals, the method comprising:

- receiving (310) the tube voltage and an emission current of the X-ray tube for the plurality of successive measurement intervals;
- dividing (320) each measurement interval into a predetermined number of time intervals at a temporal sampling frequency;
- extracting (330) a plurality of emission current samples from the received emission current at the temporal sampling frequency;
- discretizing (340) the received voltage to a finite set of discrete tube voltages;
- determining (350) a current-density-function for each discrete tube voltage assignment, which represents accumulated current-time-products of emission current samples having the respective discrete tube voltage assignment; and
- determining (360) the effective spectrum of the X-ray tube for the respective measurement interval as a function of the finite set of discrete tube voltages and the determined current-density-function for each discrete tube voltage assignment.

8. The method according to claim 7, further comprising:

- obtaining a plurality of current-density-functions for each discrete tube voltage assignment from a plurality of measurement intervals having an identical energy level;
- averaging the plurality of current-density-functions to obtain a mean effective current-density-function for the respective discrete tube voltage assignment; and
- determining the effective spectrum of the X-ray tube for the respective energy level as a function of the finite set of discrete tube voltages and the determined mean effective current-density-function for each discrete tube voltage assignment.

9. The method according to claim 7 or 8,

wherein the emission current is a known function of the tube voltage; and
wherein the current-density-function for each discrete tube voltage assignment is determined as a function of a number of occurrences of the respective discrete tube voltage assignment.

10. The method according to any one of claims 7 to 9, wherein the tube voltage is obtained at an operating point of the X-ray source at factory and/or during X-ray source conditioning.

11. An X-ray imaging method (400), comprising:

- scanning (410) an object of interest to generate X-ray image data comprising one or more image slices, each representative of a certain thickness of the object being scanned in a certain angle of projection;
- providing (420) an effective spectrum of an X-ray tube according to any one of claims 7 to 10; and
- applying (430) the effective spectrum of the X-ray tube for image reconstruction.

12. A computer program for controlling an apparatus according to any one of claims 1 to 5, which when executed by a processor is configured to carry out the method of any one of claims 7 to 10, and/or a computer program for controlling a system according to claim 6, which when executed by a processor is configured to carry out the method of claim 11.

13. A computer readable medium having stored the program element of claim 11.

Fig. 1

kVp → AD converter → Adress

**Memory for low kVp**
*mAs-density-function*

*Enabled only during low kVp Intervals*

Out → (+) ← Emission current
In

Adress →

**Memory for high kVp**
*mAs-density-function*

*Enabled only during high kVp Intervals*

Out → (+) ← Emission current
In

Fig. 2

EP 4 199 659 A1

110 _____ V(t)

10

120 _____ I(t)

## Fig. 3

210

200

100

X-ray source

V(t), I(t)

System

212

object

effective
spectrum

230

X-ray detector

reconstruction
apparatus

220

214

Image
reconstruction

Fig. 4

300

310

320

330

340

350

360

# Fig. 5

400

410

420

430

Fig. 6

Europäisches
Patentamt

European
Patent Office

Office européen
des brevets

# EUROPEAN SEARCH REPORT

Application Number

EP 21 21 4350

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/328865 A1 (ENGEL KLAUS JÜRGEN [NL] ET AL) 15 November 2018 (2018-11-15) * the whole document * | 1-13 | INV. H05G1/26 G01N23/083 A61B6/00 G06T11/00 |
| A | JP 2014 210180 A (TOSHIBA CORP; TOSHIBA MEDICAL SYS CORP) 13 November 2014 (2014-11-13) * paragraphs [[0076]], [[0077]]; figure 5 * | 2 | |

-----

-----

TECHNICAL FIELDS SEARCHED (IPC)

H05G

The present search report has been drawn up for all claims

| Place of search | Date of completion of the search | Examiner |
|---|---|---|
| Munich | 12 July 2022 | Giovanardi, Chiara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 21 21 4350**

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2022

| Patent document cited in search report | | Publication date | Patent family member(s) | | Publication date |
|---|---|---|---|---|---|
| US 2018328865 | A1 | 15-11-2018 | CN | 108603849 A | 28-09-2018 |
| | | | EP | 3384277 A1 | 10-10-2018 |
| | | | JP | 6751143 B2 | 02-09-2020 |
| | | | JP | 2019502436 A | 31-01-2019 |
| | | | US | 2018328865 A1 | 15-11-2018 |
| | | | WO | 2017093520 A1 | 08-06-2017 |
| JP 2014210180 | A | 13-11-2014 | CN | 105073010 A | 18-11-2015 |
| | | | JP | 6289223 B2 | 07-03-2018 |
| | | | JP | 2014210180 A | 13-11-2014 |
| | | | US | 2016022243 A1 | 28-01-2016 |
| | | | WO | 2014163187 A1 | 09-10-2014 |

EPO FORM P0459

For more details about this annex : see Official Journal of the European Patent Office, No. 12/82